# FASCICULE DE BREVET EUROPEEN

(11) **EP 1 214 051 B1**
(45) Date de publication et mention de la délivrance du brevet: **02.08.2006**
(21) Numéro de dépôt: 00964325.5
(22) Date de dépôt: 20.09.2000
(51) Int. Cl.: A61K 8/49, A61K 8/67, A61K 8/92, A61K 36/57, A61K 8/37, A61K 8/63, A61K 8/68, A61Q 19/00

(54) **UTILISATION D'UN PRODUIT D'HUILE VEGETALE EN TANT QU'AGENT POUR AUGMENTER LA SYNTHESE DES LIPIDES CUTANES**
VERWENDUNG VON EINEM PRODUKT AUS PFLANZLICHEM ÖL ZUR ERHÖHUNG DER HAUTLIPIDEN PRODUKTION
USE OF A VEGETABLE OIL PRODUCT AS AGENT FOR INCREASING SKIN LIPID SYNTHESIS

(30) Priorité: 22.09.1999 FR 9911844
(43) Date de publication de la demande: 19.06.2002
(60) Demande divisionnaire: 06013216.4
(73) Titulaire: Laboratoires Expanscience, 92419 Courbevoie Cedex (FR)
(72) Inventeur: MSIKA, Philippe, F-75018 Paris (FR); PICCIRILLI, Antoine, F-78000 Versailles (FR)
(74) Mandataire: Warcoin, Jacques
(86) Numéro de dépôt international: PCT/FR2000/002600
(87) Numéro de publication internationale: WO 2001/021150

(56) Documents cités:
- EP-A- 0 643 960
- EP-A- 0 775 480
- WO-A-94/21764
- WO-A-99/59523
- DE-A- 19 631 792
- FR-A- 2 187 328
- FR-A- 2 405 068
- FR-A- 2 443 835
- FR-A- 2 648 347
- FR-A- 2 653 974
- FR-A- 2 692 783
- FR-A- 2 694 692
- FR-A- 2 724 663
- FR-A- 2 762 512
- FR-A- 2 778 181
- US-A- 4 386 067

## Description

La présente invention se rapporte à l'utilisation d'un produit d'huile végétale en tant qu'agent pour augmenter la synthèse des lipides cutanés, notamment des lipides de la barrière cutanée épidermique, dans ou pour la préparation d'une composition cosmétique, pharmaceutique ou dermatologique. L'invention décrit également une méthode de traitement cosmétique à une composition cosmétique, pharmaceutique ou dermatologique pour augmenter la synthèse des lipides cutanés, notamment des lipides de la barrière cutanée épidermique, et à l'utilisation du produit végétal comme additif alimentaire.

La peau est principalement constituée de trois couches : l'épiderme, le derme et l'hypoderme.

La couche la plus externe, l'épiderme, est caractérisée par une organisation en strates correspondant à un état de différenciation croissant des kératinocytes, de la zone la plus profonde (stratum basale) à la zone la plus superficielle (stratum comeum) au sein de laquelle des éléments anuclées (cornéocytes) sont inclus dans une structure lipidique extracellulaire multilamellaire, le ciment intercornéocytaire, responsable de la fonction de barrière hydrique de la peau et de protection contre les agressions extérieures.

Les corps lamellaires ou de Oddland: secrétés par le stratum granulosum, couche intermédiaire entre le stratum basale et le stratum corneum, contiennent du cholestérol, des phospholipides et des glucosylcéramides ainsi que des hydrolysases sélectives. Ces enzymes convertissent les phospholipides et les glucosylcéramides en acides gras libres et céramides qui forment, avec le cholestérol et le sulfate de cholestérol, les bicouches lamellaires intercellulaires du stratum comeum. Les céramides participent de façon prépondérante à la formation de la barrière constitué par le stratum corneum et à la régulation des flux hydriques en solidarisant les lamelles. Une diminution importante du contenu en/et du type de céramide est notamment observée dans la dermatite atopique (ou eczéma atopique) ou dans l'acné (céramide 1) et dans les peaux sèches et prurits des personnes âgées. Le sulfate de cholestérol, via une sulfatase spécifique, est en équilibre avec le cholestérol (agent de fluidité des feuillets). Ils jouent un rôle important dans la cohésion des coméocytes et donc dans la desquamation cutanée, ainsi que dans le confort cutané.

L'altération de cette barrière cutanée provoquée par des agressions extérieures (rayonnement U.V, vent, froid, détergents etc.), par le phénomène naturel et inexorable du vieillissement et/ou par des dysfonctionnements pathologiques ou non (peaux sensibles, irritées, ou réactives) se traduit par une perturbation de l'homéostasie épidermique qu'il est souhaitable de pouvoir prévenir et/ou traiter tant sur le plan cosmétique que pharmaceutique et notamment dermatologique.

Par exemple, l'article de Ruby Ghadially et al, "Decreased Epidermal Lipid Synthesis Accounts for Altered Barrier Function in Aged Mice", The Journal Of Investigate Dennatology, Vol. 106, N°5, May 1996, enseigne qu'une barrière cutanée altérée ainsi qu'une teneur anormale en lipides dans un épiderme agé de souris peut s'expliquer par une synthèse altérée des lipides épidermiques.

Il existe donc un besoin de pouvoir stimuler la synthèse des lipides cutanés, notamment des lipides de la barrière cutanée épidermique, afin de pouvoir en particulier restaurer la fonction de barrière cutanée de l'épiderme et/ou de lutter contre les divers troubles cutanés liées à une baisse de la synthèse des lipides cutanés, notamment des lipides de la barrière cutanée épiderrnique.

On a maintenant trouvé de manière tout à fait surprenante et inattendue que l'utilisation de certains produits d'huile végétale permet d'augmenter avantageusement la synthèse des lipides cutanés, notamment des lipides de la barrière cutanée épidermique.

La présente invention concerne ainsi l'utilisation de lipides furaniques d'huile végétale en tant qu'agent pour augmenter la synthèse des lipides cutanés, notamment des lipides de la barrière cutanée épidermique connus de l'homme du métier comme ceux mentionnés ci-dessus, dans ou pour la préparation d'une composition contenant un milieu cosmétiquement, pharmaceutiquement ou dermatologiquement acceptable.

La présente invention a ainsi pour objet l'utilisation de lipides furaniques d'huile végétale pour la préparation d'une composition dermatologique destinée au traitement des peaux sensibles, des peaux irritées, des peaux réactives, des peaux atopiques, de la desquamation cutanée, du prurit, de l'ichtyose, de l'acné, de la xérose, de la dermatite atopique, ou des couches épidermiques du stratum corneum ou du granulosum de peaux ayant été soumises à un rayonnement actinique, notamment un rayonnement UV.

Les lipides cutanés sont choisis entre autres parmi les lipides épidermiques du groupe constitué par le cholestérol, le sulfate de cholestérol, les céramides 1 et 2 et les mélanges de ces derniers. Parmi les huiles végétales pouvant être utilisées, on peut citer en particulier l'huile de tournesol, de palme, de palmiste, de noix de coco, de pépins de raisins, de moutarde noire, d'ocillette, de beurre de karité, d'amande douce, de soja, d'avocat, d'arachide, de coton, de sésame, d'olive, de maïs, de cacao, de ricin, de Ben, de lin, de colza, de rocouyer, de germe de blé, de carthame, de noix, de noisettes et de navette.

Par "lipides furanique d'huile végétale" on entend selon l'invention des composés comprenant une chaîne principale linéaire hydrocarbonée en C₁₁ - C₁₉, saturée ou comprenant une ou plusieurs insaturations éthyléniques ou acétyléniques, et un groupe 2-furanylé à l'un de ses extrémités. Parmi les lipides furaniques d'huile végétale pouvant être utilisé selon l'invention, on préfère tout particulièrement les lipides furaniques de l'avocat. L'avocat comprend en effet des lipides particuliers de type furanique, dont le principal composant est un furane linoléique :

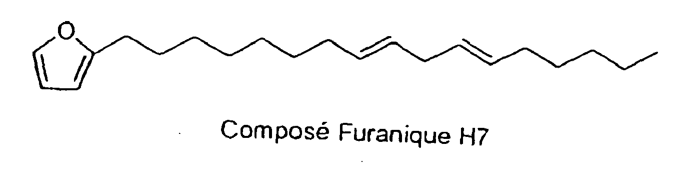

Les dérivés furaniques de l'huile d'avocat ont été décrits notamment dans Farines, M. et al, 1995, J. of Am. Oil Chem. Soc. 72, 473, et il est connu qu'ils permettent de stimuler le métabolisme de la peau et des muqueuses (EP0775480A), notamment par prolifération des cellules cutanées et par la synthèse des protéines, d'ADN et de collagène. Il est aujourd'hui bien établi que la présence de ces composés furaniques dans les feuilles ou le fruit dépend non seulement de la variété (les variétés *Hass et Fuerte* étant les plus riches en composés furaniques) mais aussi du mode d'obtention de l'huile ou d'un autre extrait végétal de l'avocat (extrait hexanique ou éthanolique des feuilles d'avocat).

En effet, on sait que ces lipides furaniques sont des métabolites de composés initialement présents dans le fruit et les feuilles qui, sous l'effet de la chaleur, se déshydratent et se cyclisent en dérivés furaniques.

Par exemple, le furane linoléique est issu de la transformation thermique du précurseur suivant :

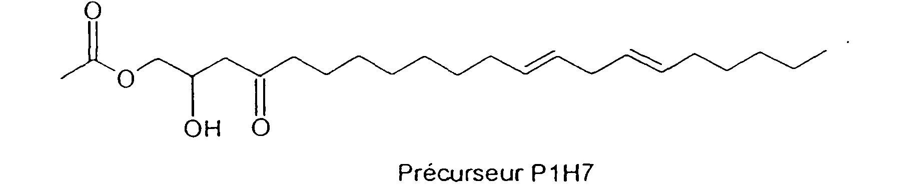

Par "lipides furaniques d'avocat", on entend en particulier selon l'invention les composants répondant à la formule : 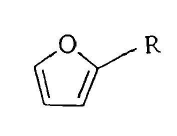 dans laquelle R est une chaîne linéaire hydrocarbonée en C₁₁ -C₁₉ de préférence C₁₃-C₁₇ saturée ou comprenant une ou plusieurs insaturations éthyléniques ou acétyléniques.

Les procédés connus pour obtenir ces composés spécifiques à partir du fruit ou de l'huile du fruit de l'avocat se résument soit à la chromatographie préparative, soit à des procédés industriels permettant d'obtenir ces lipides furaniques en mélange avec les autres composés insaponifiables d'avocat, avec une teneur maximale en lipides furaniques comprise au mieux entre 50 et environ 65% en poids seulement.

Un nouveau procédé de préparation de ces lipides furaniques de l'avocat a fait l'objet du dépôt d'une demande de brevet ce même jour. Il consiste pour l'essentiel en un procédé d'extraction sélective des lipides furaniques d'avocat, caractérisé en ce qu'il comprend les étapes consistant à préparer un insaponifiable d'avocat, puis à soumettre l'insaponifiable d'avocat à une étape de distillation moléculaire en utilisant des moyens de température réglés pour une température comprise entre 100 et 160°C et des moyens de pression réglés pour une pression comprise entre 10⁻³ et 5.10⁻² mmHg.

Cette étape de distillation moléculaire mettant en oeuvre des conditions de températures et de pressions spécifiques, constitue une caractéristique essentielle de ce procédé, en combinaison avec l'étape préalable de préparation de l'insaponifiable.

Différentes méthodes peuvent être utilisées pour concentrer la fraction insaponifiable d'une huile végétale : cristallisation par le froid, extraction liquide-liquide, distillation moléculaire.

La distillation moléculaire est particulièrement préférée, étant réalisée de préférence à une température comprise entre environ 180 et environ 260 °C en maintenant une pression comprise entre environ 10⁻³ et environ 10⁻² mmHg et de préférence de l'ordre de 10⁻³ mmHg, La concentration en insaponifiable du distillat peut atteindre 60%.

Cette distillation moléculaire, ainsi que toute autre distillation moléculaire pour la préparation des produits d'huile végétale à utiliser selon l'invention est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme du métier. Par exemple, la demande EP- 0 493 144 décrit un distillateur moléculaire de ce type. D'une manière générale, le produit à distiller est étalée en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'insaponifiable, l'avantage étant que l'huile et l'insaponifiable (ces produits étant réputés fragiles) ne sont pas dégradés au cours de l'évaporalion.

Les distillateurs moléculaires de type à film raclé, également connus de l'homme du métier, comprennent une chambre de distillation dotée d'un racleur tournant, permettant l'étalement en continu sur la surface d'évaporation (surface chaude) du produit à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile,etc.). La récupération des résidus et des distillats dans des ballons en verre, se fait par écoulement gravitationnel.

La teneur plus importante en insaponifiable dans l'huile d'avocat par rapport à d'autres huiles végétales s'explique en particulier par la présence, dans l'insaponifiable d'huile d'avocat, de constituants que l'on ne retrouve généralement pas dans l'insaponifiable de nombreuses autres huiles végétales tels que des composés furaniques et des alcools gras polyhydroxylés et qui, à eux seuls, représentent plus de 50% de l'insaponifiable. Les produits propres à cet insaponifiable d'avocat peuvent être répartis en deux fractions chimiques appelées "fraction 1" et "fraction H". Les composés actifs pour l'utilisation selon l'invention se trouvent présents dans la fraction H et ses précurseurs. La fraction H apparaît en premier lieu sur un chromatographe en phase gazeuse de l'insaponifiable d'huile d'avocat.

Selon l'invention, le produit d'huile végétale tel que décrit ci-dessus est utilisé selon une proportion comprise entre environ 0,01 et 100 % en poids, de préférence entre environ 0,5 et environ 10 % en poids, par rapport au poids total de la composition.

Le milieu cosmétiquement, pharmaceutiquement ou dermatologiquement acceptable pour l'utilisation selon l'invention peut être tout milieu adapté pour les formes galéniques connues de l'homme du métier, en vue d'une administration par voie topique, orale, entérale ou parentérale.

En particulier, ce milieu peut être une solution huileuse, une émulsion eau-dans-huile, une émulsion huile-dans eau, une microémulsion, un gel huileux, un gel anhydre, une dispersion de vésicules, de microcapsules ou de microparticules.

De préférence, la composition pour l'utilisation selon l'invention est adaptée pour une administration par application topique.

L'effet avantageux d'augmentation de la synthèse des lipides cutanés, notamment des lipides de la barrière cutanée épidermique, permet de prévenir et/ou de traiter, en d'autres termes permet le traitement des altérations de la barrière cutanée formée principalement par les couches épidermiques du stratum coméum et granulosum comme expliqué ci-dessus.

Ainsi, la composition peut-être destinée au traitement des peaux sèches et des peaux ayant été soumises à un rayonnement actinique, notamment un rayonnement U.V. tel que le rayonnement solaire ou le rayonnement d'une lampe U.V par exemple lors d'une séance de bronzage artificiel.

La composition peut également être destinée au traitement de l'ichtyose, de l'acné, de la xérose, de la dermatite atopique (ou eczéma atopique), des troubles cutanés liés à une baisse des teneurs en lipides cutanés, notamment des lipides de la barrière cutanée épidermique, des troubles de la cohésion des cornéocytes et de la desquamation cutanée, des peaux sensibles, irritées et réactives et du prurit.

La présente invention décrit également une méthode de traitement cosmétique des troubles liés au vieillissement de la peau, des muqueuses voisines et/ou des phanères, caractérisée en ce qu'on applique sur la peau, les muqueuses voisines et/ou les phanères une composition contenant au moins un produit d'huile végétale dans un milieu cosmétiquement acceptable tels que décrits ci-dessus.

L'invention décrit par ailleurs une méthode de traitement cosmétique des troubles liés au dessèchement de la peau, des muqueuses voisines et/ou des phanères, caractérisée en ce qu'on applique sur la peau, les muqueuses voisines et/ou les phanères une composition contenant au moins un produit d'huile végétale dans un milieu cosmétiquement acceptable tels que décrits ci-dessus.

L'invention décrit aussi une méthode de traitement cosmétique des troubles de la peau, des muqueuses voisines et /ou des phanères résultant d'une exposition à un rayonnement actinique, notamment un rayonnement U-V, caractérisée en ce qu'on applique sur la peau et/ou les phanères une composition contenant au moins un produit d'huile végétale dans un milieu cosmétiquement acceptable tels que décrits ci-dessus.

Dans ces méthodes de traitement cosmétiques, le produit d'huile végétale est présent dans la composition selon une proportion comprise entre environ 0,01 et 100 % en poids, de préférence entre environ 0,5 et environ 10 % en poids, par rapport au poids total de la composition.

La présente invention décrit également une composition cosmétique, pharmaceutique ou dermatologique pour augmenter la synthèse des lipides cutanés, notamment des lipides de la barrière cutanée épidermique, caractérisée en ce qu'elle comprend au moins un produit d'huile végétale dans un milieu cosmétiquement, pharmaceutiquement ou dermatologiquement acceptable, tels que décrits ci-dessus.

De préférence, dans cette composition, le produit d'huile végétale est présent dans la composition selon une proportion comprise entre environ 0,01 et 100 % en poids, de préférence entre environ 0,5 et environ 10 % en poids, par rapport au poids total de la composition.

Enfin, l'invention a encore pour objet l'utilisation d'au moins un produit d'huile végétale, tel que décrit ci-dessus, en tant qu'additif dans un aliment pour l'être humain et/ou l'animal.

Dans cette utilisation alimentaire, le produit d'huile végétale est présent dans l'aliment selon une proportion comprise entre environ 0,1 et environ 20 % en poids, par rapport au poids total de l'aliment.

Les exemples suivants sont destinés à illustrer la présente invention et ne doivent en aucun cas être interprétés comme pouvant en limiter la portée.

A moins qu'il n'en soit précisé autrement, les pourcentages indiqués dans les exemples suivants sont des pourcentages en poids.

### Exemple 1 : préparation de produits d'huile végétale et leur utilisation selon l'invention sous forme d'émulsions huiles-dans-eau

Les compositions 1.1 à 1.3 et la composition placébo suivantes ont chacune été préparées de la manière suivante :

Les composants constituant la phase aqueuse (eau et glycérine) sont placées au bain-marie à 75 °C. Les composants de la phase grasse, à l'exception du SEPIGEL 305, du SILICONE SF 1202 et du produit d'huile végétale (respectivement préparés ci-après sous les dénominations "Oléodistillat de tournesol-1 ", "Insaponifiables-1 " et "Lipides-1 "), sont placés au bain-marie à 75 °C. Juste avant de réaliser l'émulsification, on ajoute à la phase grasse le SILICONE 1202 et le produit d'huile végétale respectif. On réalise ensuite l'émulsion sous turbine à vitesse lente par incorporation de la phase grasse dans la phase aqueuse. Lorsque la préparation atteint la température de 60 °C, on ajoute le SEPIGEL 305 sous turbine à grande vitesse. On laisse ensuite refroidir la composition au repos jusqu'à température ambiante, avant son utilisation dans les essais décrits ci-après.

### 1.1) Composition Comparative 1.1 : utilisation d'un oléodistillat d'huile de tournesol

On prépare un oléodistillat de tournesol par distillation moléculaire dans un distillateur moléculaire de type centrifuge d'une huile de tournesol alimentaire du commerce. Les conditions de distillation sont les suivantes :
- température 220 °C;
- pression de 10⁻³ mmHg;
- taux de distillation : 6,7 % massique
- débit d'alimentation : 18 kg/h

Le distillat obtenu, l'oléodistillat de tournesol, présente une teneur en insaponifiable d'environ 6,2 % en poids, la partie restante étant composée par les triglycérides de l'huile de tournesol. L'Oléodistillat ainsi obtenu est dénommé "Oléodistillat de tournesol-1".

| **Composition 1.1 (formule INCI)** | **% (en poids)** |
|---|---|
| **Phase aqueuse** | |
| Eau | 67,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Oléodistillat de** tournesol-1 | 2 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

### 1.2) Composition comparative 1.2 : utilisation d'un mélange d'un insaponifiable d'avocat et de soja

On utilise le mélange d'insaponifiables d'huiles d'avocat et de soja tel que commercialisé par la société Laboratoires Pharmascience sous la dénomination "Piascledine 300® " qui consiste en un mélange de 33,3% en poids d'insaponifiable d'avocat et de 66,6% en poids d'insaponifiable de soja, par rapport au poids total du mélange (les 0,1% restants étant constitués de silice colloïdale et de butylhydroxytoluène). Ce mélange est dénommé ci-après "Insaponifiables-I".

| **Composition 1.2 (noms INCI)** | |
|---|---|
| **Phase aqueuse** | |
| Eau | 67,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Insaponifiables-1** | 2 |
| Beeswax | 3 |
| Stearate Peg 2 | 1 |
| C 12-15 Alcool Benzoate | 1 |
| Phenonip | 0,7 |
| Sepigel 305 | 0,5 |
| **TOTAL** | **100%** |

### 1.3) Composition 1.3 : utilisation de lipides furaniques d'avocat

On prépare un insaponifiable d'avocat comme décrit dans le brevet FR-2 678 632. Sa composition est la suivante :
- alcools gras polyhydroxylés 24,3 %
- lipides furaniques 55,5 %
- stérols 3,1 %
- squalène 1,4 %
- autres 15,7 % (1)
   (1) acides gras libres, hydrocarbures, tocophérols, cétones grasses et pigments lourds

On soumet cet insaponifiable à une distillation moléculaire à l'aide du distillateur moléculaire à film raclé commercialisé par la société Leybold sous la dénomination "KDL4". Les conditions de distillation sont les suivantes :
- température surface chaude: 108°C
- pression : 10⁻³ mm Hg
- vitesse de rotation de l'arbre: 240 t/min.
- débit d'insaponifiable d'avocat: 400 ml/h
Rendement en distillat: 48,6 %
Composition du distillat :
- alcools gras Polyhydroxylés : n.m.
- lipides furaniques 99,1 %
- stérols n.m.
- squalène n.m.
- autres 0,9%(1)
(1) acides gras libres, hydrocarbures et cétones grasses
("n.m." : non mesurable, c'est à dire une teneur inférieure à 0,05 %)
Il s'agit donc d'un distillat très riche en lipides furaniques dans la mesure où la teneur de ces dernier excède 99 %. Ce distillat est dénommé par la suite "Lipides-1 ".

| **Composition 1.3 (noms INCI)** | **% (en poids)** |
|---|---|
| **Phase aqueuse** | |
| Eau | 69 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Lipides-1** | 0,3 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

### 1.4) Composition placébo

| **Composition placébo (noms INCI)** | **% (en poids)** |
|---|---|
| **Phase aqueuse** | |
| Eau | 69,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

### Exemple 2 : évaluation in vitro de l'effet des compositions 1.1, 1.2, 1.3, et placébo sur le métabolisme des lipides épidermiques dans un modèle organotypique de peau humaine entière en culture

Dans ce qui suit, on utilise les abréviations suivantes:
EGF : facteur de croissance épidermique, *Epidermal growth factor;*
CCM : chromatographie en couche mince;
MCF : milieu de culture des disques de peau humaine;
MIF : milieu d'incubation des disques de peau humaine;
PBS : tampon phosphate salin, *phosphate buffered saline.*

L'objet de cette étude est d'étudier l'effet des quatre compositions 1.1, 1.2, 1.3 et placébo décrites ci-dessus sur le métabolisme des lipides épidermiques.

L'étude est réalisée *in vitro* dans un modèle organotypique de peau humaine entière en culture. Deux techniques mises en oeuvre successivement :
- mesure de l'incorporation d'acétate radiomarqué au carbone 14 dans le *totum* des lipides épidermiques néosynthétisés ;
- analyse en chromatographie en couche mince pour séparer les principales classes de lipides épidermiques radiomarqués néosynthétisés.

L'effet des produits à l'essai est comparé à celui observé en présence du facteur de croissance épidermique (EGF), dilué dans le milieu de culture des disques de peau humaine, et en présence d'une formulation cosmétique commercialisée contenant de l'acide lactique. L'EGF et l'acide lactique stimulent tous deux de manière connue la synthèse des céramides par les kératinocytes (Ponec M. Gibbs S., Weerheim A., Kempenaar J., Mulder A. and Mommaas A.M. - Epidermal growth factor and temperature regulate keratinocytes differentiation - Arch. Dermatol. Res., 1997, 289, 317-326; et Rawlings A.V., Davies A., Carlomusto M., Pillai S. Ahang K., Kosturbo R., Verdejo P., Feinberg C., Nguyen L. and Chandar P. - Effect of lactic acid isomers on keratinocyte ceramide synthesis, stratum corneum lipid levels and stratum corneum barrier function - Arch. Dermatol. Res., 1996, 288, 383-390).

### 1) Matériels et méthode

### 1.1) Produits à l'essai, produits de référence et réactifs

Les compositions 1.1, 1.2, 1.3, et placébo ont été préparées comme décrit ci-dessus. L'EGF provenait de chez R&D SYSTEMS. La formulation cosmétique contenant de l'acide lactique, appelée par la suite "acide lactique", a été achetée dans le réseau de distribution grandes surfaces.

La solution de rinçage des disques de peau humaine après l'incubation est le tampon PBS : NaCl 8g/l ; Na₂HPO₄ 1,15g/l ; KH₂PO₄ 0,2g/l ; KCl 0,2g/l ; CaCl₂ 0,1g/l ; MgCl₂ 0,1 g/l ; pH 7,4.

Les autres réactifs, de qualité analytique, proviennent de chez CARLO ERBA, GIBCO et SIGMA, sauf indication contraire.

### 1.2) Système d'essai

Un fragment de peau humaine a été collecté après une opération de plastie abdominale. Celle-ci a été réalisée chez une femme âgée de 24 ans (sujet I0129). Des disques de peau de 8 mm de diamètre ont été découpés à l'aide d'un emporte-pièce.

Les disques de peau sont déposés dans des nacelles. Les nacelles sont placées dans des puits de culture contenant le milieu MCF, composé du milieu MEM/M199 (3/4, 1/4 ; v/v) additionné de pénicilline (50 UI/ml), de streptomycine (50 µg/ml), de bicarbonate de sodium (0,2 %, p/v) et de SVF (2 %, v/v).

### 1.3) Incubation des produits à l'essai et des produits de référence avec le système d'essai

Les produits à l'essai sont testés non dilués. Ils sont déposés au centre de chaque disque de peau humaine, à raison de 10 mg/cm². Le milieu d'incubation des disques de peau humaine (milieu MIF) est composé du milieu MCF contenant 1 µCi/ml d'acétate marqué au carbone 14 (AMERSHAM, activité spécifique : 57 mCi/mmole).

L'EGF est testé à 10 ng/ml dans le milieu MIF. L'acide lactique est utilisée en application topique (10 mg/cm²).

Les disques de peau humaine sont incubés en présence des produits à l'essai et des produits de référence pendant 18 heures à 37°C dans une atmosphère humide contenant 5% de CO₂.

Des disques de peau témoins sont incubés en parallèle en absence de produits à l'essai et de produits de référence.

Chaque condition expérimentale est réalisée en *quadriplicate.*

L'échelle de temps suivante est utilisée : 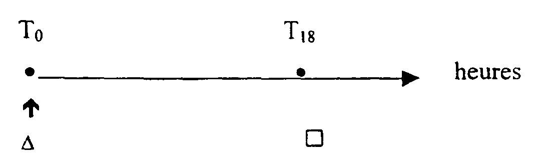
↑ : application topique des produits à l'essai et du produit de référence et dilution de l'EGF dans le milieu d'incubation des disques de peau
Δ : ajout d'acétate marqué au carbone 14 dans le milieu de culture
□ : dissociation derme/épiderme et évaluation des effets

### 1.4) Evaluation des effets

### 1.4.1) Néosynthèse des lipides épidermiques totaux

A la fin de l'incubation, les disques de peau humaine sont abondamment rincés avec le tampon PBS. L'épiderme de chaque disque de peau est dissocié du derme par un choc thermique contrôlé (eau MilliQ, 2 min, 62°C). Les épidermes ainsi dissociés sont digérés par la trypsine (ICN, 1%, p/v) pendant une nuit à 37°C. La dissociation cellulaire est facilitée par action des ultrasons.

Les lipides néosynthétisés, marqués au carbone 14, sont extraits par partition entre une phase organique (méthanol/chloroforme, 1/4, v/v) et une phase aqueuse (chlorure de potassium à 0,25 M). La phase organique est évaporée sous azote et les résidus sont repris dans un_mélange chloroforme/méthanol, 2/1 (v/v).

La radioactivité de chaque échantillon, correspondant à la quantité d'acétate incorporé dans les lipides néosynthétisés, est mesurée en scintillation liquide.

Les résultats sont exprimés en cpm/mg d'épiderme.

### 1.4.2) Nature des lipides épidermiques néosynthétisés

A partir des échantillons de lipides extraits, des aliquotes de 20µl, correspondant à 3700 cpm, sont déposées sur des plaques de chromatographie de silice 60 (MERCK). Celles-ci sont développées dans trois solvants successifs :
- chloroforme/acétone/méthanol, 38/2/10 (v/v/v),
- chloroforme/acétone/méthanol, 40/5/5 (v/v/v),
- chloroforme/acétate d'éthyl/éther/méthanol, 36/10/3/1 (v/v/v/v).

Ce système permet de séparer le sulfate de cholestérol, les cérébrosides, les céramides, le cholestérol et les tri- + di-glycérides. Les lipides plus polaires, appelés par la suite "lipides polaires", restent au point de dépôt.

Les plaques de silice sont ensuite mises en exposition avec des films pour autoradiographie pendant 15 jours (AMERSHAM, Hyperfilm beta max).

La position des différentes classes de lipides- lipides polaires, sulfate de cholestérol, cérébrosides, céramides 1 et 2, cholestérol et tri- + di-glycérides - est déterminée à l'aide des standards appropriés.

La radioactivité des spots séparés et révélés grâce à l'autoradiographie est comptée avec un analyseur de radioactivité argon-méthane sur couche mince (BERTHOLD). Les résultats sont exprimés en pourcentages de la radioactivité des lipides totaux néosynthétisés et déposés sur la CCM.

### 1.5) Traitement des données

Les groupes de données (groupe témoin et groupes traités) sont comparés par une analyse de la variance à un facteur (ANOVA 1, p<0,05), suivie par un test de Dunnett.

### 2) Résultats

Après une nuit d'incubation en présence des disques de peau humaine, les compositions à l'essai et les compositions de référence n'ont pas d'effet significatif sur la néosynthèse des lipides épidermiques totaux (tableau 3.1). En revanche, ils modifient de façon notable la proportion des différents lipides épidermiques dans ce *totum :*
- l'**EGF** à 10 ng/ml diminuait de 46% la néosynthèse du sulfate de cholestérol et augmentait de 55% la néosynthèse du céramide 2 (tableau 3.2);
- l'**acide lactique** augmente d'un facteur 1,59 la néosynthèse des cérébrosides (tableau 3.2);
- la **composition 1.1** augmente d'un facteur 2,26 et 4,61 la néosynthèse respective des céramides 1 et 2, d'un facteur 5,04 la néosynthèse du cholestérol. Il diminue de 73% la néosynthèse des tri- et di-glycérides (tableau 3.3);
- la **composition 1.2** augmente d'un facteur 1,32 la néosynthèse du sulfate de cholestérol, d'un facteur 2,47 et 2,51 la néosynthèse respective des céramides 1 et 2, d'un facteur 4,62 la néosynthèse du cholestérol. Il diminue de 77% la néosynthèse des tri- et di-glycérides (tableau 3.2);
- la **composition 1.3** augmente d'un facteur 1,24 la néosynthèse du sulfate de cholestérol, d'un facteur 1,59 et 3,66 la néosynthèse respective des céramides 1 et 2, d'un facteur 4,14 la néosynthèse du cholestérol. Il diminue de 84% la néosynthèse des tri- et di-glycérides (tableau 3.2);
- la **composition placébo** diminue de 59% la néosynthèse des tri- et di-glycérides (tableau 3.3.) sans provoquer d'augmentation significative des différents lipides épidermiques analysés.

En conclusion, dans les conditions expérimentales retenues, les composition 1.1, 1.2, 1.3, et placébo n'ont pas d'effet significatif sur la néosynthèse des lipides épidermiques totaux.

Ils modifient par contre de façon significative la proportion des différentes classes des lipides épidermiques séparés en chromatographie en couche mince dans le *totum.*

Ils diminuent la néosynthèse des tri- et di-glycérides au profit des autres lipides épidermiques.

La composition 1.1 augmente la néosynthèse du cholestérol (sans modification de la néosynthèse du sulfate de cholestérol) et la néosynthèse des céramides.

Les compositions 1.2 et 1.3 augmentent la néosynthèse du sulfate de cholestérol et du cholestérol, et la néosynthèse des céramides.

La composition placébo ne provoque pas d'augmentation significative des différents lipides épidermiques analysés.

### 3) Tableaux de résultats

**Tableau 3.1) : Effet des compositions 1.1, 1.2, 1.3, et placebo ainsi que de l'EGF et d'une formulation cosmétique contenant de l'acide lactique sur la néosynthèse des lipides épidermiques totaux dans des disques de peau humaine entière, après 18 heures d'incubation**

| Témoin | EGF 10 ng/ml | Acide lactique | Composition 1.2 | Composition 1.3 | Composition 1.1 | Composition placébo |
|---|---|---|---|---|---|---|
| 4183,35 | 3104,63 | 1864,31 | 2479,26 | 3646,42 | 873,10 | 1506,12 |
| 4407,89 | 5043,66 | 4919,12 | 3858,70 | 5255,70 | 3726,00 | 4154,88 |
| 3691,69 | 3606,76 | 4027,67 | 6571,13 | 3900,53 | 3802,63 | 4429,96 |
| 1062,72 | 4565,02 | 1076,21 | 2457,78 | 3209,16 | 905,55 | 2373,94 |
| **3336,41** | **4080,02** | **2971,83** | **3841,72** | **4002,95** | **2326,82** | **3116,23** |
| +/- | +/- | +/- | +/- | +/- | +/- | +/- |
| **1545,02** | **883,02** | **1800,62** | **1934,04** | **882,63** | **1660,22** | **1408,09** |
| *100* | *122* | *89* | *115* | *120* | 70 | *93* |

| | | | | | | |
|---|---|---|---|---|---|---|
| Les résultats sont exprimés en cpm/mg d'épiderme. | | | | | | |
| En gras : moyenne et écart type | | | | | | |
| En italique : pourcentage du groupe témoin | | | | | | |
| * : moyenne significativement différente du groupe témoin (p<0,05) | | | | | | |

**Tableau 3.2) : Effet des compositions 1.2 et 1.3 ainsi que de l'EGF et d'une formulation cosmétique contenant de l'acide lactique sur la néosynthèse des lipides polaires, du sulfate de cholestérol, des cérébrosides, des céramides 1 et 2, du cholestérol et des tri- + di-glycérides dans des disques de peau humaine entière, après 18 heures d'incubation**

| Produit | Lipides polaires | Sulfate de cholestérol | Cérébrosides | Céramide 1 | Céramide 2 | Cholestérol | Tri- + di-glycérides |
|---|---|---|---|---|---|---|---|
| | 28,94 | 6,04 | 1,89 | 2,03 | 2,03 | 8,23 | 50,83 |
| | 24,96 | 4,80 | 2,08 | 2,67 | 2,07 | 5,04 | 53,76 |
| | 31,74 | 6,26 | 4,02 | 2,71 | 2,15 | 7,89 | 45,23 |
| Témoin | 42,90 | 5,12 | 4,44 | 1,67 | 1,47 | 5,61 | 38,84 |
| | **32,14** | **5,56** | **3,11** | **2,27** | **1,93** | **6,69** | **47,17** |
| | +/- | +/- | +/- | +/- | +/- | +/- | +/- |
| | **7,70** | **0,71** | **1,31** | **0,51** | **0,31** | **1,60** | **6,58** |
| | 100 | 100 | 100 | 100 | 100 | 100 | 100 |
| | 57,27 | 2,31 | 2,87 | 2,02 | 3,81 | 7,07 | 42,67 |
| | 34,42 | 3,69 | 3,82 | 1,41 | 2,38 | 9,46 | 44,82 |
| | 27,69 | 3,44 | 4,77 | 3,41 | 2,74 | 7,86 | 50,08 |
| EGF | 33,53 | 2,57 | 4,02 | 4,02 | 3,05 | 9,99 | 42,83 |
| 10 ng/ml | **38,23** | **3,00*** | **3,87** | **2,72** | **3,00*** | **8,60** | **45,10** |
| | +/- | +/- | +/- | +/- | +/- | +/- | +/- |
| | **13,04** | **0,67** | **0,78** | **1,21** | **0,61** | **1,36** | **3,46** |
| | 119 | 54 | 125 | 120 | 155 | 128 | 96 |
| | 49,20 | 4,07 | 4,46 | 2,37 | 1,70 | 7,77 | 33,43 |
| | 33,04 | 4,16 | 4,54 | 2,64 | 3,32 | 9,72 | 42,58 |
| | 64,49 | 5,90 | 6,07 | 2,94 | 2,57 | 8,07 | 31,95 |
| Acide | 53,51 | 4,25 | 4,68 | 2,58 | 1,44 | 8,67 | 24,86 |
| Lactique | **50,06** | **4,60** | **4,94*** | **2,63** | **2,26** | **8,56** | **33,21** |
| | +/- | +/- | +/- | +/- | +/- | +/- | +/- |
| | **13,05** | **0,87** | **0,76** | **0,24** | **0,86** | **0,86** | **7,28** |
| | 156 | 83 | 159 | 116 | 117 | 128 | 70 |
| | 24,57 | 6,89 | 4,41 | 2,78 | 6,86 | 44,35 | 10.13 |
| | 28,71 | 8,34 | 5,91 | 5,94 | 3,34 | 35,04 | 12,72 |
| Composition | 33,09 | 6,86 | 4,02 | 4,55 | 4,79 | 36,97 | 9,72 |
| 1.2 | 35,78 | 7,35 | 7,76 | 9,20 | 4,37 | 34,01 | 11,52 |
| | **30,54** | **7,36*** | **5,53** | **5,62*** | **4,84*** | **37,59*** | **11,02*** |
| | +/- | +/- | +/- | +/- | +/- | +/- | +/- |
| | **4,93** | **0,69** | **1,70** | **2,72** | **1,48** | **4,67** | **1,37** |
| | 95 | 132 | 178 | 247 | 251 | 562 | 23 |
| | 38,88 | 6,23 | 6,23 | 3,20 | 8,92 | 28,63 | 7,92 |
| | 43,70 | 8,25 | 4,55 | 3,51 | 7,25 | 24,34 | 8,41 |
| Composition | 37,70 | 6,88 | 3,97 | 3,52 | 6,69 | 33,55 | 7,68 |
| 1.3 | 47,94 | 6,15 | 6,13 | 4,24 | 5,40 | 24,30 | 5,85 |
| | **42,06** | **6,88*** | **5,22** | **3,62*** | **7,07*** | **27,71*** | **7,47*** |
| | +/- | +/- | +/- | +/- | +/- | +/- | +/- |
| | **4,70** | **0,97** | **1,13** | **0,44** | **1,46** | **4,39** | **1,12** |
| | 131 | 124 | 168 | 159 | 366 | 414 | 16 |

| | | | | | | | |
|---|---|---|---|---|---|---|---|
| Les résultats sont exprimés en pourcentage des lipides totaux radiomarqués déposés sur la CCM. | | | | | | | |
| En gras : moyenne et écart type | | | | | | | |
| En italique : pourcentage du groupe témoin | | | | | | | |
| * : moyenne significativement différente du groupe témoin (p<0,05) | | | | | | | |

**Tableau 3.3) : Effet des compositions 1.1 et palcébo sur la néosynthèse des lipides polaires, du sulfate de cholestérol, des cérébrosides, des céramides 1 et 2, du cholestérol et des tri- + di-glycérides dans des disques de peau humaine entière, après 18 heures d'incubation**

| Produit | Lipides polaires | Sulfate de cholestérol | Cérébrosides | Céramide 1 1 | Céramide 2 | Cholestérol | Tri- + diglycérides |
|---|---|---|---|---|---|---|---|
| | 28.94 | 6.04 | 1.89 | 2.03 | 2.03 | 8.23 | 50.83 |
| | 24,96 | 4,80 | 2.08 | 2.67 | 2.07 | 5,04 | 53.76 |
| | 31.74 | 6.26 | 4.02 | 2,71 | 2,15 | 7,89 | 45.23 |
| Témoin | 42.90 | 5,12 | 4,44 | 1,67 | 1,47 | 5,61 | 38,84 |
| | **32,14** | **5,56** | **3,11** | **2,27** | **1,93** | **6,69** | **47,17** |
| | +/- | +/- | +/- | +/- | +/- | +/- | +/- |
| | **7,70** | **0,71** | **1,31** | **0,51** | **0,31** | **1,60** | **6,58** |
| | 100 | 100 | 100 | 100 | 100 | 100 | 100 |
| | 37.24 | 6.43 | 4.35 | 5.12 | 8.38 | 32.19 | 16.28 |
| | 32,48 | 4.83 | 5,45 | 4,22 | 7,77 | 32,51 | 12.74 |
| Composition | 31.92 | 3.81 | 5.68 | 5.43 | 8.06 | 32.23 | 10.86 |
| 1.1 | 25.87 | 4.41 | 4.49 | 5,75 | 11.37 | 38.00 | 10,11 |
| | **31.88** | **4.87** | **4.99** | **5.13*** | **8.90*** | **33.73*** | **12,50*** |
| | +/- | +/- | +/- | +/- | +/- | +/- | +/- |
| | **4.66** | **1.12** | **0.67** | **0.66** | **1.67** | **2.85** | **2.75** |
| | 99 | 88 | 161 | 226 | 461 | 504 | 26 |
| | 73.67 | 9.27 | 7.69 | 2.44 | 2.05 | 3.03 | 18.60 |
| | 58.70 | 11.03 | 7.60 | 2.51 | 3.39 | 3.71 | 13.07 |
| Composition | 55,90 | 6.63 | 3,71 | 4.21 | 3,67 | 8,66 | 17.20 |
| placébo | 63.39 | 5.69 | 4.48 | 4.17 | 3.35 | 4.93 | 27,98 |
| | **62,92** | **8,16** | **5,87** | **3,33** | **3,12** | **5,08** | **19,21 *** |
| | +/- | +/- | +/- | +/- | +/- | +/- | +/- |
| | **7,81** | **2,44** | **2,07** | **0,99** | **0,72** | **2,51** | **6,30** |
| | 196 | 147 | 189 | 147 | 161 | 76 | 41 |

| | | | | | | | |
|---|---|---|---|---|---|---|---|
| Les résultats sont exprimés en pourcentage des lipides totaux radiomarqués déposés sur la CCM. | | | | | | | |
| En gras : moyenne et écart type | | | | | | | |
| En italique : pourcentage du groupe témoin | | | | | | | |
| * : moyenne significativement différente du groupe témoin (P<0,05) | | | | | | | |

## Revendications

1. Utilisation de lipides furaniques d'huile végétale pour la préparation d'une composition dermatologique destinée au traitement des peaux sensibles, des peaux irritées, des peaux réactives, des peaux atopiques, de la desquamation cutanée, du prurit, de l'ichtyose, de l'acné, de la xérose, de la dermatite atopique, ou des couches épidermiques du stratum corneum ou du granulosum de peaux ayant été soumises à un rayonnement actinique, notamment un rayonnement UV.

2. Utilisation selon la revendication 1, **caractérisée en ce que** les lipides furaniques d'huile végétale sont des lipides furaniques de l'avocat.

3. Utilisation selon la revendication 1 ou 2, **caractérisée en ce que** la composition est destinée à une application topique.

4. Utilisation selon la revendication 1 ou 2, **caractérisée en ce que** 1a composition est destinée à une administration orale.

## Claims

1. Use of furan lipids from plant oil for the preparation of a dermatological composition for treating sensitive skin, irritated skin, reactive skin, atopic skin, cutaneous desquamation, pruritus, ichthyosis, acne, xerosis, atopic dermatitis, or the epidermal layers of the stratum corneum or of the granulosum of skin that have been subjected to actinic radiation, especially UV radiation.

2. Use according to Claim 1, **characterized in that** the furan lipids from plant oil are furan lipids from avocado.

3. Use according to Claim 1 or 2, **characterized in that** the composition is for topical application.

4. Use according to Claim 1 or 2, **characterized in that** the composition is for oral administration.

## Patentansprüche

1. Verwendung von Furanyllipiden aus Pflanzenöl für die Herstellung der dermatologischen Zusammensetzung, die zur Behandlung von empfindlicher Haut, gereizter Haut, reagierender Haut, atopischer Haut, Hautabschuppung, Hautjucken, Ichthyose, Akne, Xerosis, atopischer Dermatitis oder Epidermisschichten des Stratum corneum oder des Granulosums von Haut, die aktinische Bestrahlung unterzogen worden ist, insbesondere UV-Bestrahlung, bestimmt ist.

2. Verwendung nach Anspruch 1, **dadurch gekennzeichnet, dass** die Furanyllipide von Pflanzenöl Furanyllipide der Avocado sind.

3. Verwendung nach Anspruch 1 oder 2, **dadurch gekennzeichnet, dass** die Zusammensetzung für eine topische Anwendung bestimmt ist.

4. Verwendung nach Anspruch 1 oder 2, **dadurch gekennzeichnet, dass** die Zusammensetzung für eine orale Verabreichung bestimmt ist.
